# FASCICULE DE BREVET EUROPEEN

(11) **EP 0 737 062 B1**
(45) Date de publication et mention de la délivrance du brevet: **06.05.1998**
(21) Numéro de dépôt: 95904527.9
(22) Date de dépôt: 22.12.1994
(51) Int. Cl.: A61K 9/107, A61K 47/00

(54) **LOTION EAU DANS HUILE CONTENANT DU CORTICOSTEROIDE**
WASSER-IN-OEL-LOTION, DIE EIN CORTICOSTEROID ENTHAELT
WATER-IN-OIL LOTION CONTAINING A CORTICOSTEROID

(30) Priorité: 27.12.1993 DE 4344697
(43) Date de publication de la demande: 16.10.1996
(73) Titulaire: GALDERMA S.A., CH-6330 Cham (CH)
(72) Inventeur: SATTLER, Henning, D-22297 Hamburg (DE)
(74) Mandataire: Tezier Herman, Béatrice
(86) Numéro de dépôt international: EP9404288
(87) Numéro de publication internationale: WO9517883

(56) Documents cités:
- DATABASE WPI Week 8946 Derwent Publications Ltd., London, GB; AN 83-805363 & JP,A,58 162 516 (FUJISAWA PHARM.) , 23 Octobre 1989
- CUTIS, vol. 50,no. 5, 1992 pages 383-386, K.L. WATSKY ET AL. 'Water-in-oil emollients as steroid-sparing adjunctive therapy in the treatment of psoriasis.'

## Description

La présente invention concerne un nouveau type de lotion comprenant comme matière active un corticostéroïde.

On utilise souvent des crèmes comme véhicules pour la thérapie avec des corticostéroïdes. Dans le cas de ces crèmes, il s'agit de systèmes d'émulsion soit huile dans eau, soit eau dans huile.

On connaît par exemple du document EP-A-0 217 146 une crème huile dans eau comprenant du diester de l'hydrocortisone. Mais les crèmes huile dans eau jouent un rôle mineur dans le domaine de la thérapie avec des corticostéroïdes, car la plupart des processus inflammatoires surgissent en cas de peaux sèches. Ainsi, les émulsions du type huile dans eau (contenant de l'eau dans la phase extérieure de l'émulsion) ne s'y prêtent que de manière limitée.

On utilise plus souvent les crèmes de type eau dans huile (contenant de l'huile dans la phase extérieure de l'émulsion). Du fait de leur consistance épaisse et par conséquent de leur propriété à se laisser difficilement étaler, elles présentent l'inconvénient d'être inappropriées à l'application sur des surfaces étendues. Il s'y ajoute qu'elles laissent un film onctueux sur la peau, ce qui est défavorable du point de vue cosmétique. Une crème à base d'une émulsion eau dans huile comprenant comme matière active des diesters d'hydrocortisone est par exemple décrite dans le document EP-A-0 217 141 ; des crèmes "stabilisées" (émulsions eau dans huile) comprenant des corticostéroïdes sont décrites par exemple dans le document US 4 284 630.

Ces deux inconvénients -mauvaise aptitude à l'application sur des surfaces étendues et non-acceptation cosmétique- s'appliquent également et à un plus haut degré à un troisième type de préparations topiques, à savoir aux crèmes telles que les pommades sur base de vaseline/paraffine (pommades grasses). A ces inconvénients s'y ajoute l'encrassement du linge et des vêtements en contact avec la peau traitée avec ces préparations.

Comme quatrième type de véhicule pour la thérapie avec des corticostéroïdes peuvent être considérés les systèmes d'émulsion liquides du type huile dans eau, donc les lotions. Leur champ d'application ressemble à celui des crèmes huile dans eau. Les lotions huile dans eau présentent cependant le même inconvénient fondamental que les crèmes huile dans eau. Elles ne se prêtent pas aux peaux sèches que l'on rencontre le plus souvent dans ce domaine d'indication. Par conséquent, le champ d'application de ces lotions est très limité, malgré leur aptitude générale à l'application sur des surfaces étendues.

Les teintures et les teintures solidifiées par des agents gélificateurs constituent un cinquième type de base pour les corticostéroïdes. Dans ce cas là, il s'agit de solutions, le plus souvent à base d'éthanol, d'isopropanol/propylèneglycol ou de polyéthylèneglycol/eau. Elles sont souvent qualifiées (par erreur) de lotions, bien qu'elles ne représentent pas de systèmes d'émulsion. Si ces lotions contiennent en plus une substance tensioactive, celle-ci ne sert qu'à la solubilisation du corticostéroïde peu soluble. Une telle teinture est par exemple décrite (comme "lotion") dans le document EP-A-0 292 893.

Il s'ensuit de ce qui précède qu'il n'existe pas jusqu'à ce jour de véhicules pour les corticostéroïdes répondant simultanément aux exigences suivantes :
- aptitude aux peaux sèches ;
- aptitude à l'application sur des surfaces étendues ;
- acceptation dermatologique.

Ce sont exclusivement les lotions du type eau dans huile qui se prêtent à ces fins. Jusqu'à présent, elles ne sont pas connues dans la thérapie avec les corticostéroïdes. Même dans le secteur cosmétique, les lotions de ce type eau dans huile n'existent que depuis peu de temps. La raison en est que ce type de lotion est difficile à stabiliser. Les lotions eau dans huile ont tendance à se séparer, pendant le stockage, en phase huileuse et en phase aqueuse. Par conséquent, elles ne peuvent pas être réalisées en "diluant" une crème eau dans huile avec de l'eau. Pour obtenir une lotion du type eau dans huile, il faut une combinaison compliquée de plusieurs émulsionnants avec des composants lipophiles particulièrement adaptés.

Il est nécessaire d'avoir recours à des combinaisons d'émulsionnants parce qu'il n'est guère possible de réaliser une lotion eau dans huile stable sur la base d'un seul des émulsionnants ci-dessous indiqués. Les émulsionnants eau dans huile (ou leurs mélanges) représentent des types nouveaux, particulièrement bien adaptés à des lotions eau dans huile. Ils ne sont pas identiques aux émulsionnants utilisés couramment pour les crèmes eau dans huile, comme par exemple les sorbitanéoléates et les sorbitanestéarates (produits Span ®). Ces derniers (voir US 4 284 630) ne se prêtent qu'à la préparation de crèmes eau dans huile.

En ce qui concerne un telle lotion eau dans huile comprenant comme matière active un corticostéroïde, il faut en outre tenir compte du fait que la plupart des émulsionnants et stabilisants pouvant être pris en considération pour les lotions eau dans huile ont tendance à décomposer le corticostéroïde contenu. La stabilité de stockage de la matière active nécessaire pour un produit thérapeutique est donc mise en cause. Il est entendu par "stabilité de stockage nécessaire", que la lotion eau dans huile ainsi que la matière active présentent la conservabilité requise pour un médicament, donc au moins 3 ans, et ce également pour des températures élevées (supérieures à 30° C).

On connaît du document DE-A-28 26 257 le corticostéroïde hydrocortisone-21-acétate-17-alpha-propionate.

L'invention a pour but de proposer une lotion eau dans huile contenant de l'hydrocortisone-21-acétate-17-alpha-propionate, présentant une bonne stabilité de l'émulsion liquide, assurant en même temps une bonne conservabilité de la matière active pendant le stockage et se prêtant de façon optimale à la thérapie avec les corticostéroïdes dans les cas de peaux sèches et d'une application sur des surfaces étendues.

Ce but est atteint par des lotions eau dans huile ayant les compositions A, B, C ou D suivantes avec les combinaisons d'émulsionnants eau dans huile a) à d),

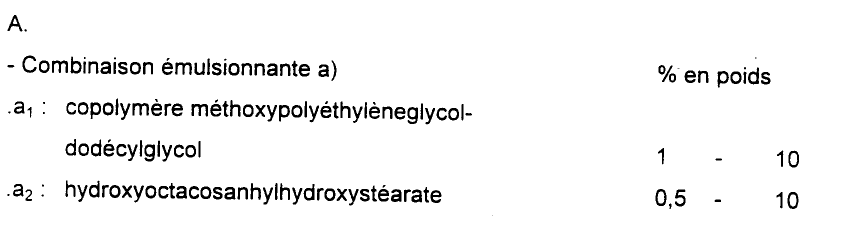

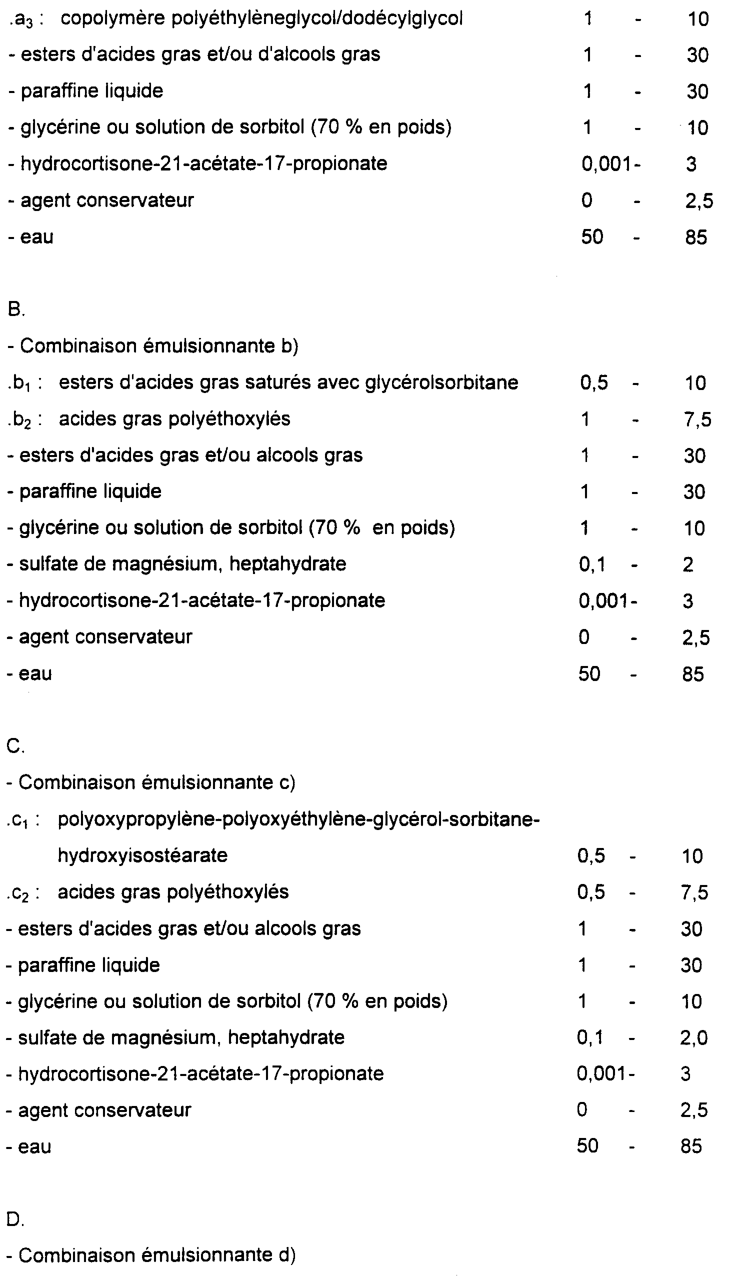

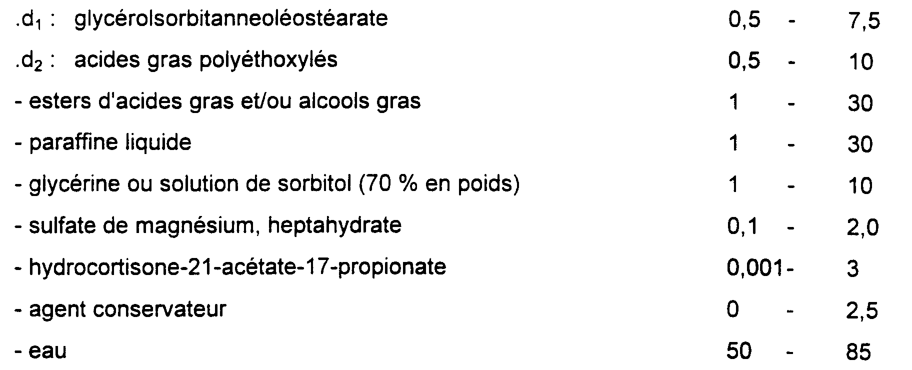

chacune de ces compositions A à D pouvant comprendre, le cas échéant, des additifs, et, outre la combinaison émulsionnante indiquée, un ou plusieurs des autres composants mentionnés a₁ à d₂ ou une ou plusieurs des combinaisons émulsionnantes a) à d) ci-dessus cités.

Dans les lotions selon les compositions A, B, C ou D, les combinaisons émulsionnante eau dans huile a) à d) sont formées par les émulsionnants, stabilisants, épaississants et systèmes complexes émulsionnants a₁, a₂ etc. à d₁, d₂ en tant que composants.

Le composant a₁ est de préférence un émulsionnant eau dans huile non-ionique comprenant environ 22 motifs d'éthylèneglycol. De tels émulsionnants sont commercialisés sous la dénomination Elfacos E 200 (Akzo Chemie, NL).

Le composant a₂ agit en tant que régulateur de consistance. De tels produits sont commercialisés sous la dénomination Elfacos C 26 (Akzo Chemie, NL).

Le composant a₃ est de préférence un émulsionnant eau dans huile non-ionique ayant environ 20 à 45 motifs d'éthylèneglycol et une valeur HLB d'environ 7. De tels émulsionnants sont commercialisés sous les dénominations Elfacos ST 9 et Elfacos ST 37 (Akzo Chemie, NL) ; en outre, ils sont décrits dans la littérature ci-dessus indiquée.

Le composant b₁ est un émulsionnant eau dans huile non-ionique, ayant de préférence un poids moléculaire d'environ 630, une valeur HLB d'environ 4,5 et une consistance cireuse. De tels produits sont notamment commercialisés sous la dénomination Arlacel 986 (ICI Europa Ltd., Belgique).

Le composant b₂ est de préférence un émulsionnant eau dans huile saturé non-ionique ayant un poids moléculaire d'environ 1245 et une valeur HLB d'environ 6,4. De tels produits sont notamment commercialisés sous la dénomination Arlacel 989 (ICI) ou Cremophor WO 7 (BASF).

Le composant b₂ correspond aux composants c₂ et d₂.

Le composant c₁ est un émulsionnant eau dans huile saturé non-ionique. De tels produits sont également commercialisés sous la dénomination Arlacel 780 (ICI).

Les esters d'acides gras préférés sont l'isopropylpalmitate, l'isopropylmyristate, l'isopropylisostéarate, le myristylemyristate, le 2-cétylhexylpalmitate (Cegesoft. Soc. Henkel), l'isooctylstéarate (Cetiol 868, Soc. Henkel), l'ester décylique de l'acide oléique (Cetiol V) et l'ester cétylique de l'acide palmitique (Cutina CP, Soc. Henkel).

Les alcools gras préférés sont l'acool myristique, l'alcool cétylique, l'alcool stéarique, l'alcool cétostéarylique, l'alcool béhénylique et le 2-octyldodécanol (Eutanol G).

Comme paraffine on utilise de préférence une paraffine très liquide, le cas échéant en combinaison avec une paraffine semi-solide (vaseline), une paraffine solide ou des microcires. On préfère particulièrement les paraffines linéaires et ramifiées.

Une solution de sorbitol appropriée est par exemple une solution aqueuse de 70 %. De tels produits sont notamment commercialisés sous les dénominations "Karion F", "Karion F flüssig SK" ou "Karion F flüssig kosmetik" (E. Merck, Allemagne).

Les lotions contiennent de préférence l'hydrocortisone-21-acétate-17-propionate dans une concentration de 0,001 à 1 % en poids, en particulier de 0,01 à 0,3 % en poids, toujours par rapport au poids total.

On préfère particulièrement l'hydrocortisone-21-acétate-17-alpha-propionate dans une quantité de 0,0025-0,2 % en poids, en particulier d'environ 0,13 % en poids, toujours par rapport au poids total de la lotion.

On préfère particulièrement les lotions comprenant les constituants et, le cas échéant, les additifs suivants :

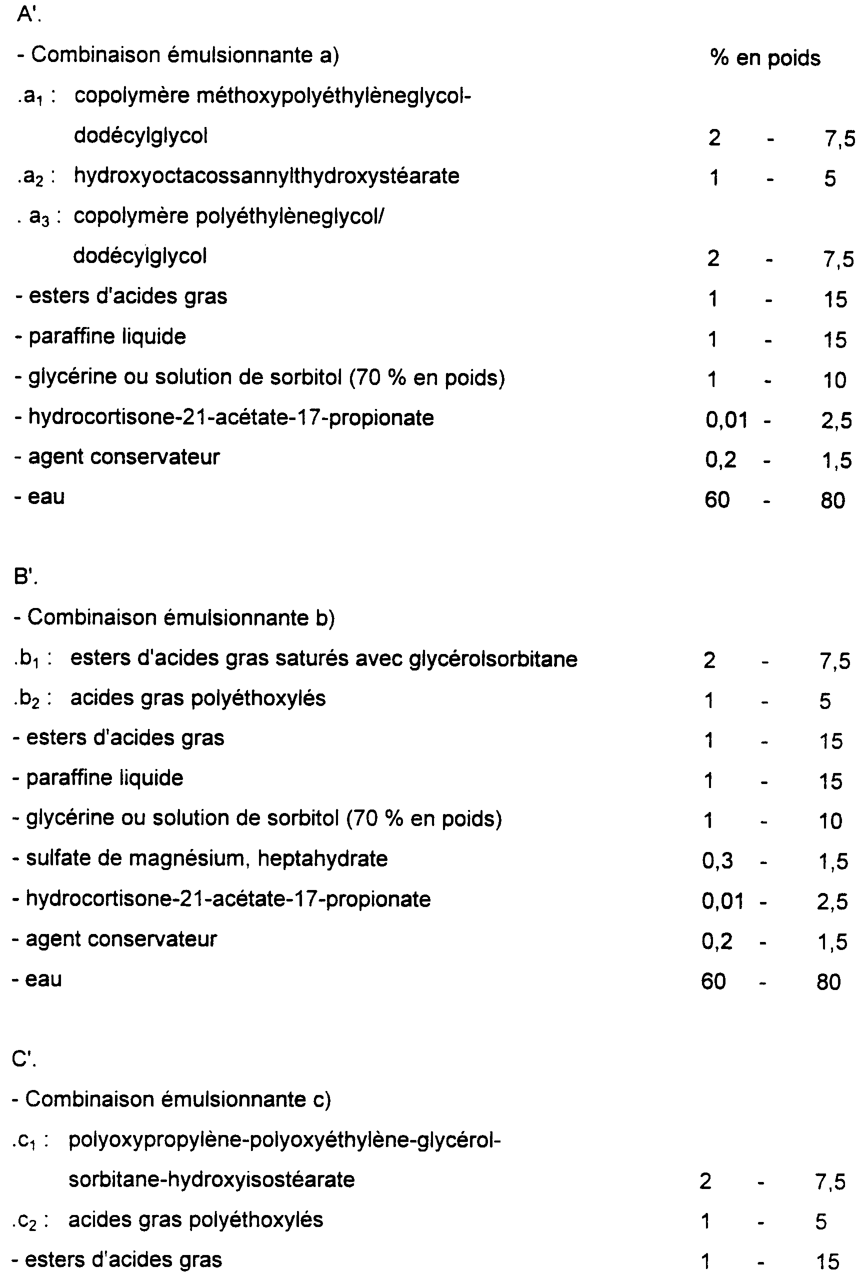

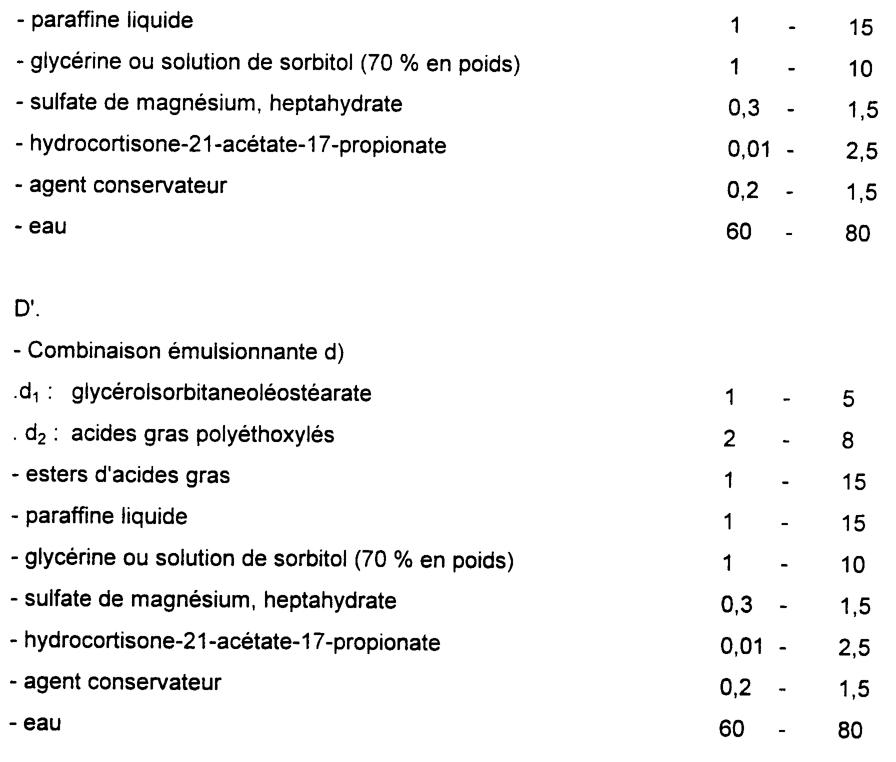

Afin d'ajuster le pH à une valeur préférée de 4 à 5, la lotion selon l'invention peut contenir des additifs acides en faibles quantités, par exemple d'environ 0,01 - 0, 5 % en poids. A cet effet, les acides carboxyliques comme l'acide succinique, l'acide lactique, l'acide citrique ou également l'acide phosphorique ainsi que leurs sels sont bien appropriés en tant que tampons.

Il est également possible d'ajouter des agents conservateurs et des stabilisants. Parmi les matières appropriées, on compte par exemple le parabène, les chlorures de benzalkonium, le p-chloro-m-crésol, l'acide benzoïque, l'acide sorbique, le phénoxyéthanol, l'alcool benzylique, le buthylhydroxyanisol et le butylhydroxytoluène, le tocopherolacétate, l'ascorbylpalmitate ainsi que leurs mélanges. Ces matières peuvent être contenues dans une quantité de 0,01 à 2,5 % en poids.

Les émulsionnants appropriés aux lotions eau dans huile contenant les corticostéroïdes représentent des types ayant en partie des compositions très complexes avec de faibles valeurs HLB ainsi que leurs mélanges. La sélection des émulsionnants a été effectuée dans le but d'obtenir une lotion eau dans huile stable pendant plusieurs années - et ce également à des températures élevées -et compatible avec les matières actives respectivement d'obtenir une stabilisation des matières actives pendant la période indiquée.

La phase grasse de la lotion eau dans huile se compose par exemple d'un mélange équilibré de paraffines liquides à chaîne linéaire ou ramifiée, d'esters naturels et synthétiques, d'acides gras à chaîne linéaire ou ramifiée, saturés ou non saturés avec des alcools monovalents ou polyvalents (par exemple l'isopropylpalmitate, l'isopropylmyristate, l'isopropylstéarate, l'isopropylisostéarate, le myristylmyristate, le cétylpalmitate, l'oléyloléate, les triglycérides à chaîne moyenne, l'huile d'arachide, l'huile d'olive, l'huile de ricin, l'huile de ricin hydrogénée), d'acides gras (par exemple l'acide linoléique, l'acide oléique) ainsi que d'alcools gras (par exemple l'alcool myristique, l'alcool cétostéarylique), de l'octyldodécanol et du squalène, dans une quantité de1 à 30 % en poids, par exemple.

Les lotions selon l'invention peuvent contenir d'autres additifs.

La phase aqueuse contient par exemple des alcools polyvalents (par exemple la glycérine, le propylèneglycol, le 1,3-butylèneglycol, des polyéthylèneglycols), des alcools saccharidiques (par exemple le sorbitol, le xylitol), des lécithines (par exemple la lécithine de soja, l'ovolécithine) ou des composés du type phosphatidylcholine, dipalmitoylphosphatidylcholine, par exemple dans une quantité de 1 à 10 % en poids.

Avec la nouvelle lotion selon l'invention, on propose une nouvelle lotion eau dans huile contenant de l'hydrocrotisone-17-propionate-21-acétate se distinguant par une bonne activité, une bonne acceptation dermatologique et une stabilité de stockage élevée.

En comparaison avec une crème eau dans huile, la lotion eau dans huile présente le grand avantage d'être rapidement absorbée par la peau et de ne pas laisser de film onctueux gênant -contrairement à la crème, malgré le même type d'émulsion.

Pour la préparation de la lotion, les constituants de la phase grasse comme la paraffine, les esters isopropyliques d'acides gras et la combinaison d'émulsionnants sont fondus et portés à une température élevée, comprise par exemple entre 60 et 80°, de manière connue en soi. Le sorbitol, la glycérine et le sulfate de magnésium ainsi que les constituants de la phase aqueuse sont dissouts dans l'eau à une température élevée. Les phases sont réunies et émulsionnées. La matière active est ajoutée à une température plus basse, comprise par exemple entre 40 et 60°. Ensuite, on laisse refroidir la masse sous agitation.

La matière active est très bien absorbée à partir de la lotion selon l'invention qui présente une stabilité de stockage excellente. Elle est par exemple utilisée pour le traitement des eczémas, des dermatitides, du psoriasis ainsi que des inflammations.

Afin de guérir ou traiter ces maladies, la lotion selon l'invention peut être appliquée de façon topique sur les parties lesées. La quantité appliquée de la lotion varie en fonction de la concentration de la matière active. En général, une quantité appropriée est appliquée à la partie lesée plusieurs fois par jour, selon la gravité de la maladie à traiter.

Sauf indication contraire, toutes les quantités, parties et pourcentages s'appliquent par rapport au poids et à la quantité totale ou au poids total des préparations.

Les exemples suivants ont pour but d'illustrer la présente invention, mais n'ont aucun caractère limitatif.

La préparation des lotions eau dans huile se fait de la manière suivante : les constituants de la phase grasse, comme la paraffine, les esters d'acides gras et la combinaison de l'émulsionnant sont chauffés à environ 80° C dans un mélangeur-homogénisateur pouvant être mis sous vide. Ensuite, les constituants de la phase aqueuse, comme le sorbitol, la glycérine et le sulfate de magnésium ainsi que les acides, les agents tampon sont dissouts dans une quantité correspondante en eau à une température d'environ 80° C et la phase aqueuse est ajoutée à la phase grasse dans le mélangeur. Sous vide, le mélange est refroidi à environ 40° C et homogénéisé sous agitation. A cette température, on ajoute la matière active et on refroidit la préparation à environ 25° C sous agitation et sous vide. Les agents conservateurs sont dissouts -en fonction de leurs propriétés physiques respectivement chimiques- soit dans la phase grasse à une température d'environ 80° C, soit dans la phase aqueuse à environ 80° C ou ajoutés à la préparation à une température d'environ 40° C.

Dans les exemples suivants, on utilise comme corticostéroïde l'hydrocortisone-21-acétate-17-propionate :

### EXEMPLE 1 :

On prépare une lotion eau dans huile en mettant en oeuvre les constituants ci-dessous indiqués :

| | % en poids |
|---|---|
| copolymère méthoxypolyéthylèneglycol-dodécylglycol (Elfacos E 200) | 3,0 |
| hydroxyoctacosanylhydroxystéarate (Elfacos C 26) | 2,5 |
| copolymère polyéthylèneglycol/dodécylglycol (Elfacos ST 9) | 5,0 |
| isopropylpalmitate | 11,0 |
| squalène synthétique | 2,0 |
| paraffine liquide | 10,0 |
| sorbitol 70 % (Karlon F) | 1,0 |
| hydrocortisone-21-acétate-17-propionate | 0,200 |
| buthylhydroxytoluène | 0,05 |
| alcool benzylique | 0,5 |
| phénoxyéthanol | 0,5 |
| acide citrique | q.s.* |
| eau déminéralisée | ad 100,00 |

| | |
|---|---|
| (* pour ajuster le pH à 4-5) | |

La préparation se fait de la manière ci-dessus indiquée. L'agent conservateur, le phénoxyéthanol, est dissout dans la phase aqueuse, l'antioxidant, le butylhydroxytoluène, est dissout dans la phase grasse. La matière active, l'hydrocortisoneacéponate, est dissoute dans l'alcool benzylique et ajoutée à la lotion à une température d'environ 40° C.

### EXEMPLE 2

On prépare une lotion eau dans huile en mettan en oeuvre les constituants ci-dessous indiqués :

| | % en poids |
|---|---|
| ester d'acide gras saturé avec glycérolsorbitane (Arlacel 986) | 1,5 |
| acides gras polyéthoxylés (Arlacel 989 ou Cremophor WO 7) | 3,0 |
| isopropylmyristate | 5,0 |
| paraffine liquide | 11,0 |
| 1,3-butylène-glycol | 3,0 |
| sulfate de magnésium, heptahydrate | 0,5 |
| hydrocortisone-21-acétate-17-propionate | 0,127 |
| alcool benzylique | 1,0 |
| eau déminéralisée | ad 100,0 |

### EXEMPLE 3

On prépare une lotion eau dans huile en mettant en oeuvre les constituants ci-dessous indiqués :

| | % en poids |
|---|---|
| polyoxypropylène-polyoxyéthylène-glycérol-sorbitanehydroxyisostéarate (Arlacel 780) | 4,0 |
| acides gras polyéthoxylés (Arlacel 989) | 2,0 |
| isopropylmyristate | 7,0 |
| octyldodécanol | 5,0 |
| paraffine liquide | 13,5 |
| glycérine | 2,5 |
| sorbitol 70 % (Karion F) | 2,5 |
| hydrocortisone-21-acétate-17-propionate | 0,127 |
| phosphatidylcholine | 3,0 |
| chlorure de benzalkonium | 0,25 |
| acide lactique | q.s.* |
| eau déminéralisée | ad 100,0 |

| | |
|---|---|
| (* pour ajuster le pH à 4-5) | |

### EXEMPLE 4

| | % en poids |
|---|---|
| glycérol-sorbitaneoléostéarate (Arlacel 481, Soc. ICI) | 2,0 |
| acides gras polyéthoxylés (Arlacel 989, Soc. ICI) | 6,0 |
| décyloléate (Cétiol V, Soc. Henkel) | 15,0 |
| paraffine à chaîne ramifiée (Arlamol HD, Soc. ICI) | 8,0 |
| microcire | 1,0 |
| glycérine | 4,0 |
| sulfate de magnésium, heptahydrate | 0,70 |
| sel de sodium de l'acide sorbique | 0,20 |
| hydrocortisone-21-acétate-17-propionate | 0,05 |
| hydrogénocitrate de diammonium | 0,10 |
| acide citrique | q.s.* |
| eau déminéralisée | ad 100,00 |

| | |
|---|---|
| (* pour ajuster le PH à 4-5) | |

## Revendications

1. Lotions eau dans huile contenant de l'hydrocortisone-21-acétate-17-propionate ayant les compositions A, B, C ou D suivantes, avec les combinaisons d'émulsionnants eau dans huile a) à d) 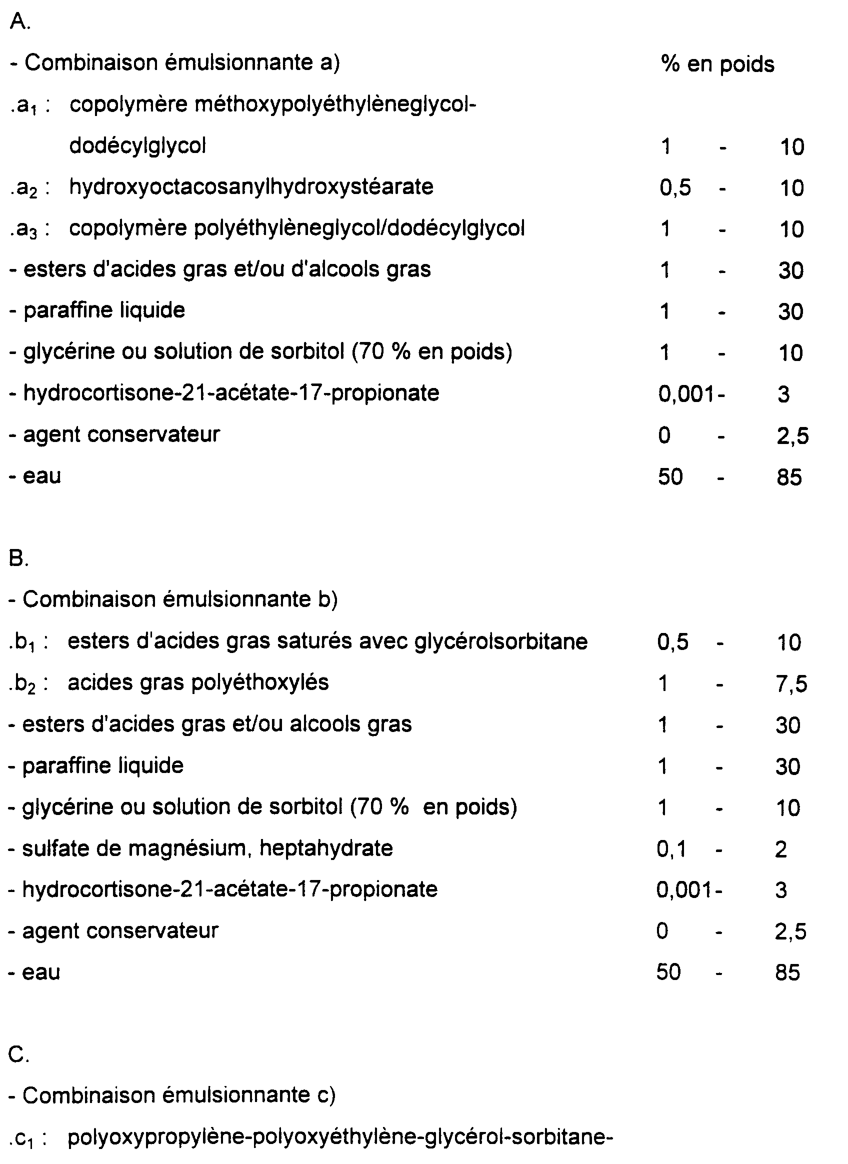 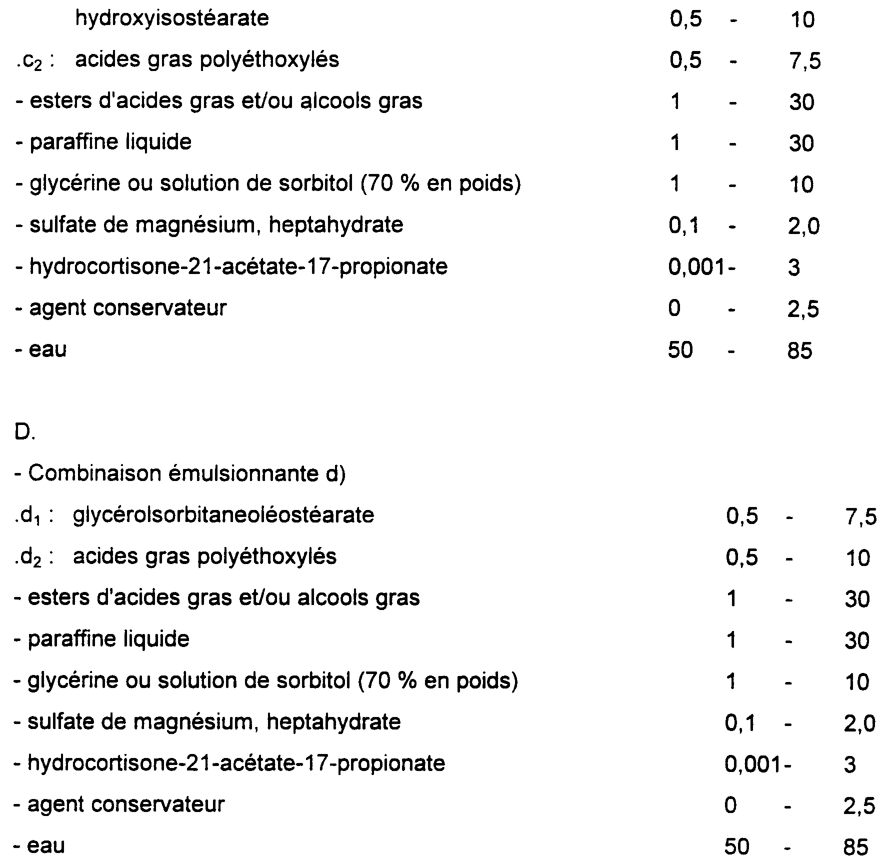 chacune de ces compositions A à D pouvant comprendre, le cas échéant, des additifs, et, outre la combinaison émulsionnante indiquée, un ou plusieurs des autres composants mentionnés a₁ à d₂ ou une ou plusieurs des combinaisons émulsionnantes a) à d) ci-dessus cités.

2. Lotions selon la revendication 1, comprenant les constituants et, le cas échéant, les additifs suivants :

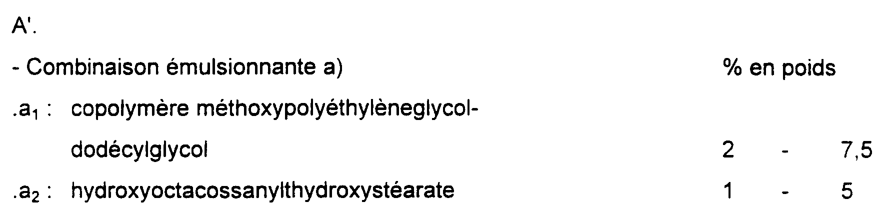

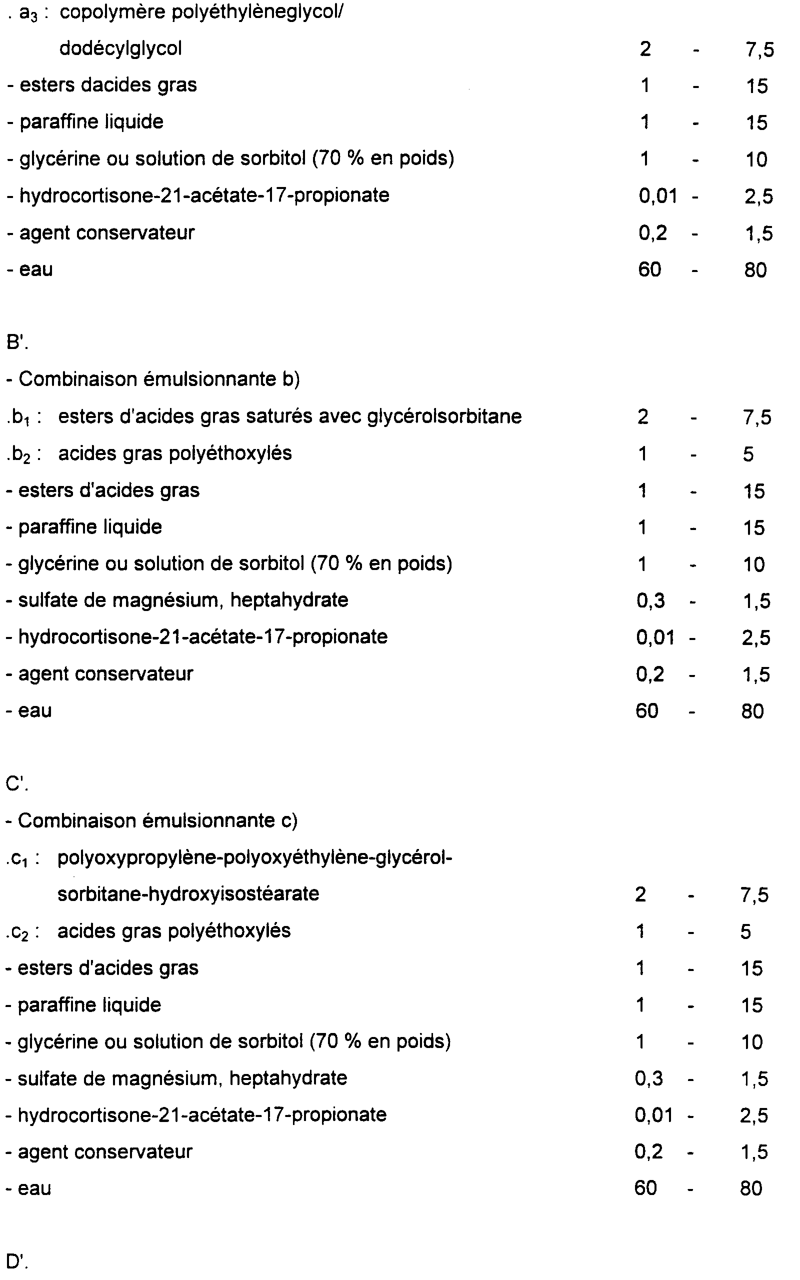

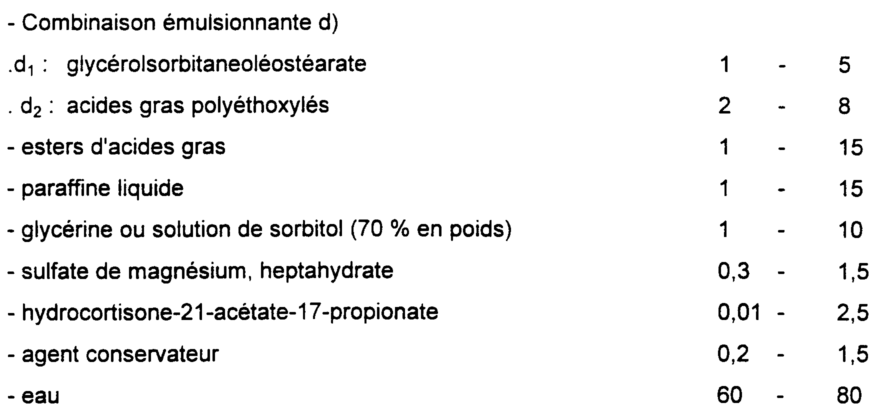

3. Lotions selon les revendications 1 ou 2, caractérisées en ce qu'elles contiennent de 0,0025 à 0,2 % en poids de l'hydrocortisone-21-acétate-17-alpha-propionate (hydrocortisoneacéponate).

4. Lotions selon la revendication 1 présentant la composition suivante :
| | % en poids |
|---|---|
| ester d'acide gras saturé avec glycérolsorbitane (Arlacel 986) | 1,5 |
| acides gras polyéthoxylés (Arlacel 989 ou Cremophor WO 7) | 3,0 |
| isopropylmyristate | 5,0 |
| paraffine liquide | 11,0 |
| 1,3-butylène-glycol | 3,0 |
| sulfate de magnésium, heptahydrate | 0,5 |
| hydrocortisone-21-acétate-17-propionate | 0,127 |
| alcool benzylique | 1,0 |
| eau déminéralisée | ad 100,0 |

## Claims

1. Water-in-oil lotions containing hydrocortisone-21-acetate-17-propionate, having the compositions A, B, C or D below, with the water-in-oil emulsifying combinations a) to d) 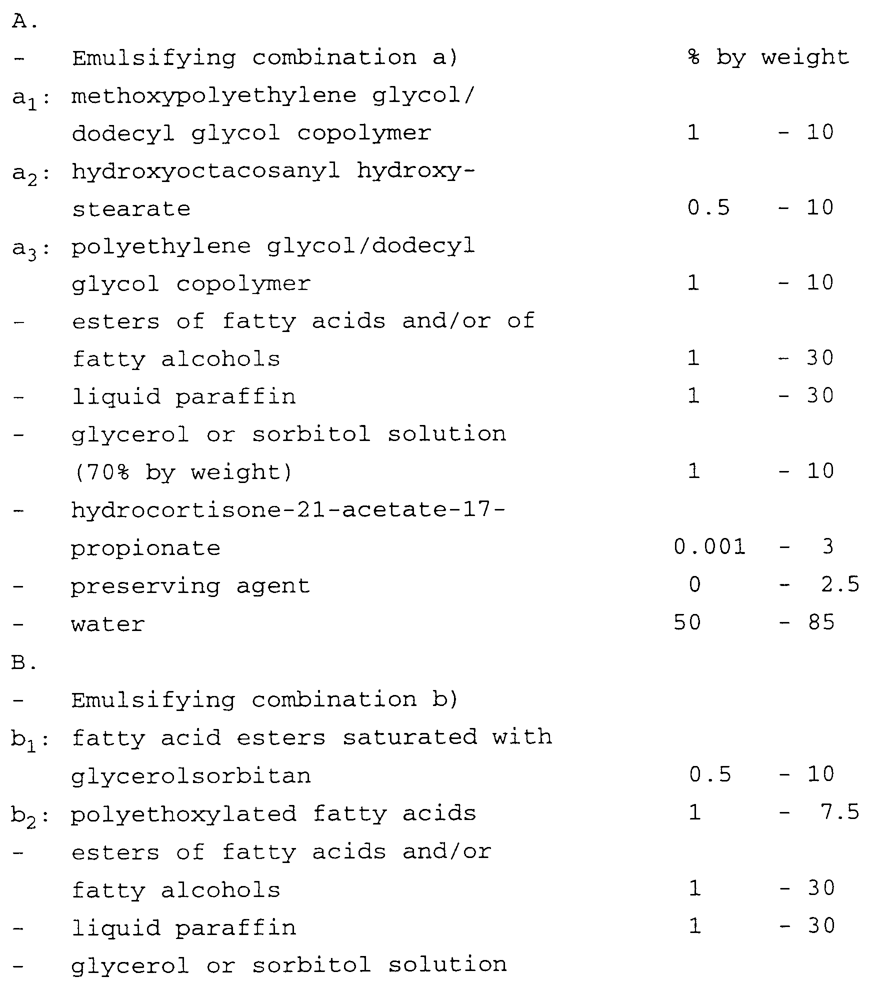 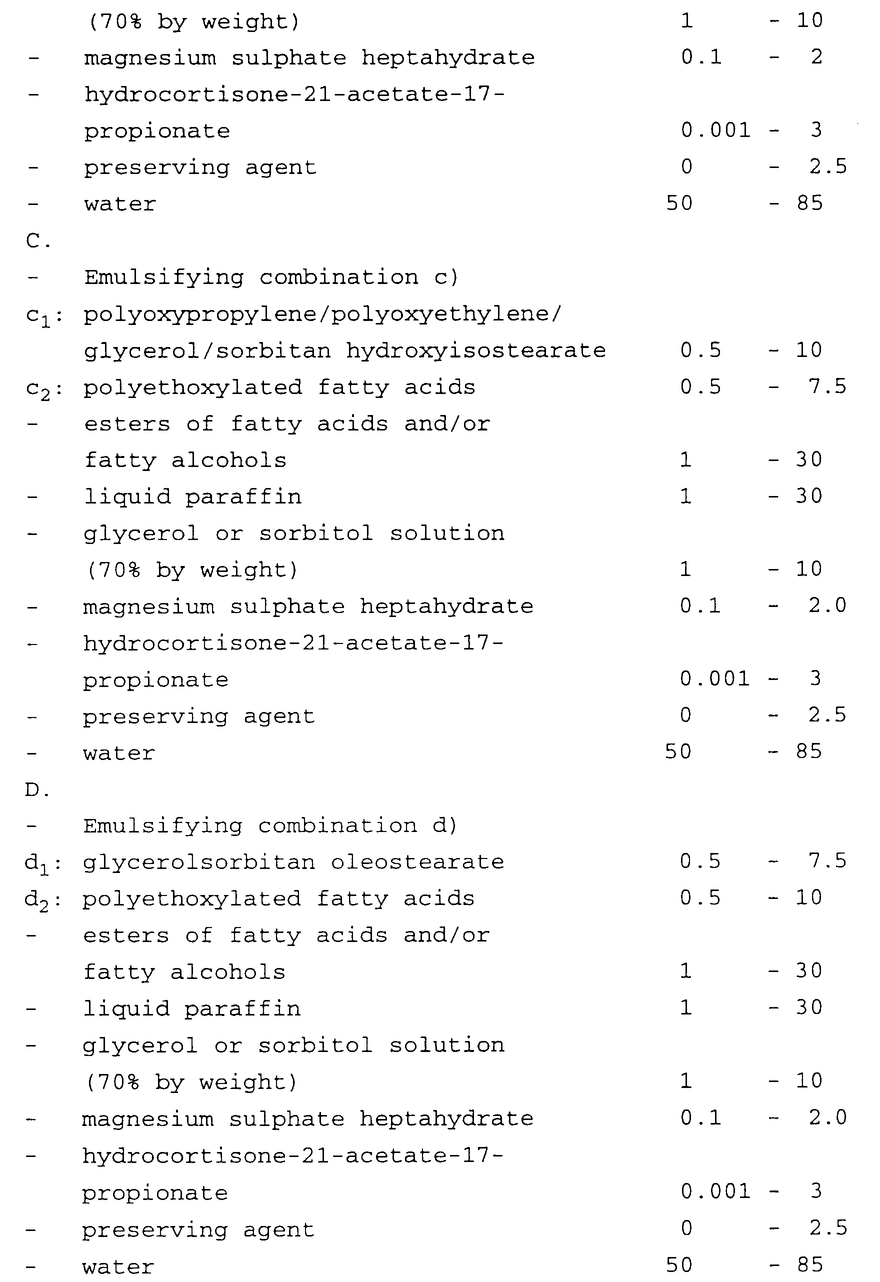 it being possible for each of these compositions A to D to comprise, where appropriate, additives and, besides the emulsifying combination indicated, one or more of the other components mentioned a₁ to d₂ or one or more of the emulsifying combinations a) to d) mentioned above.

2. Lotions according to Claim 1, comprising the constituents and, where appropriate, the additives below:

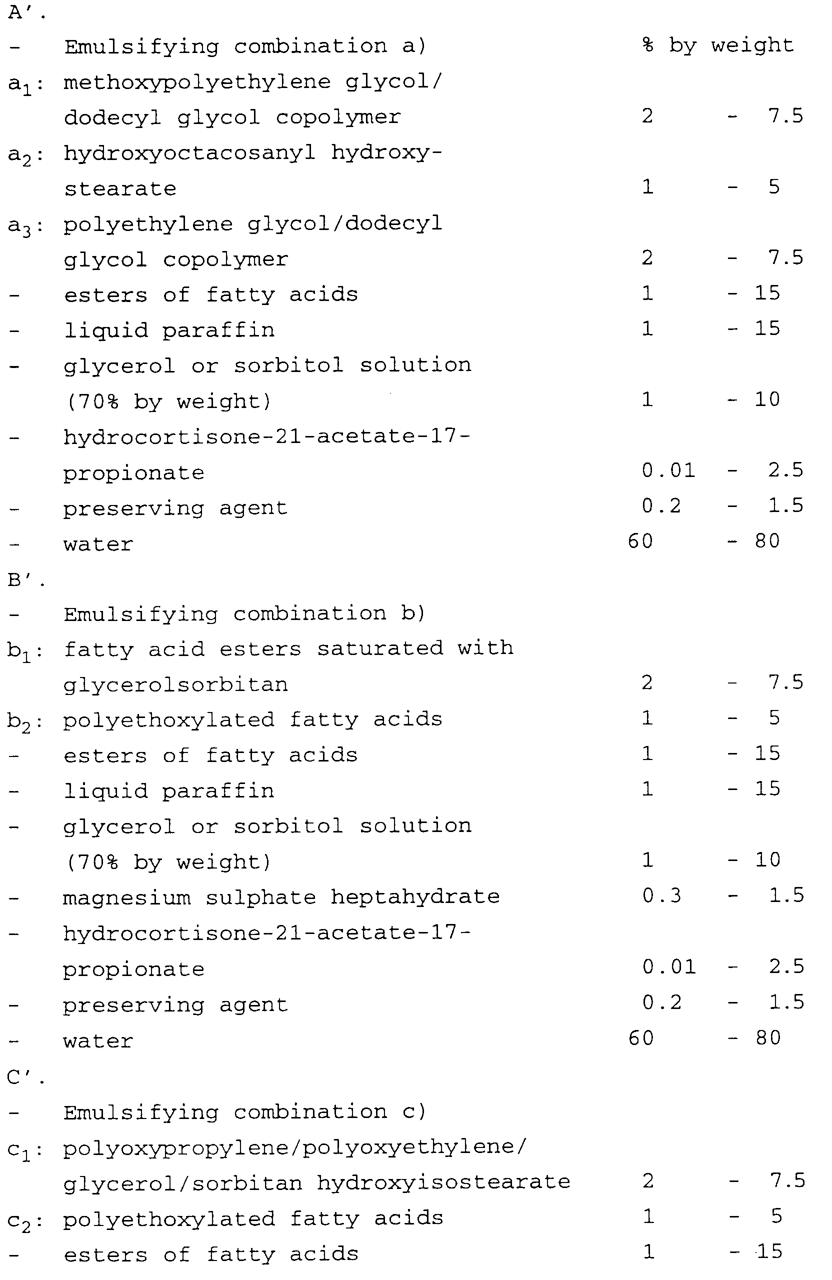

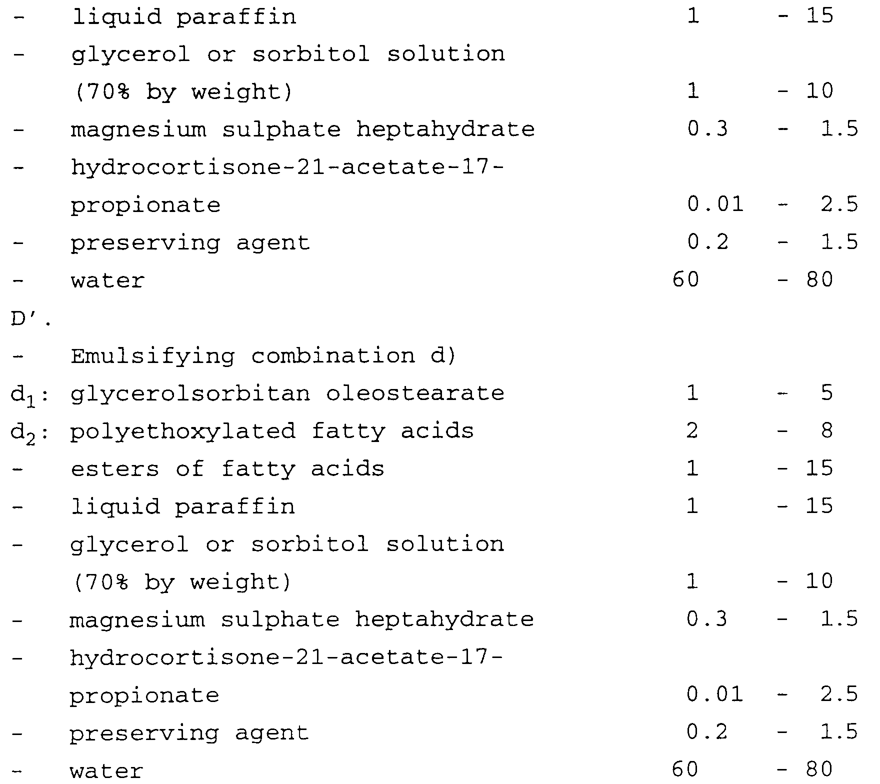

3. Lotions according to Claim 1 or 2, characterized in that they contain from 0.0025 to 0.2% by weight of hydrocortisone-21-acetate-17-alpha-propionate (hydrocortisoneaceponate).

4. Lotions according to Claim 1, having the following composition:
| | % by weight |
|---|---|
| fatty acid ester saturated with glycerol sorbitan (Arlacel 986) | 1.5 |
| polyethoxylated fatty acids (Arlacel 989 or Cremophor WO 7) | 3.0 |
| isopropyl myristate | 5.0 |
| liquid paraffin | 11.0 |
| 1,3-butylene glycol | 3.0 |
| magnesium sulphate heptahydrate | 0.5 |
| hydrocortisone-21-acetate-17-propionate | 0.127 |
| benzyl alcohol | 1.0 |
| demineralized water | qs 100.0 |

## Patentansprüche

1. Wasser-in-Öl-Lotionen, die Hydrocortison-21-acetat-17-propionat enthalten und die folgenden Zusammensetzungen A, B, C oder D mit den Kombinationen von Wasser-in-Öl-Emulgatoren a) bis d) aufweisen: 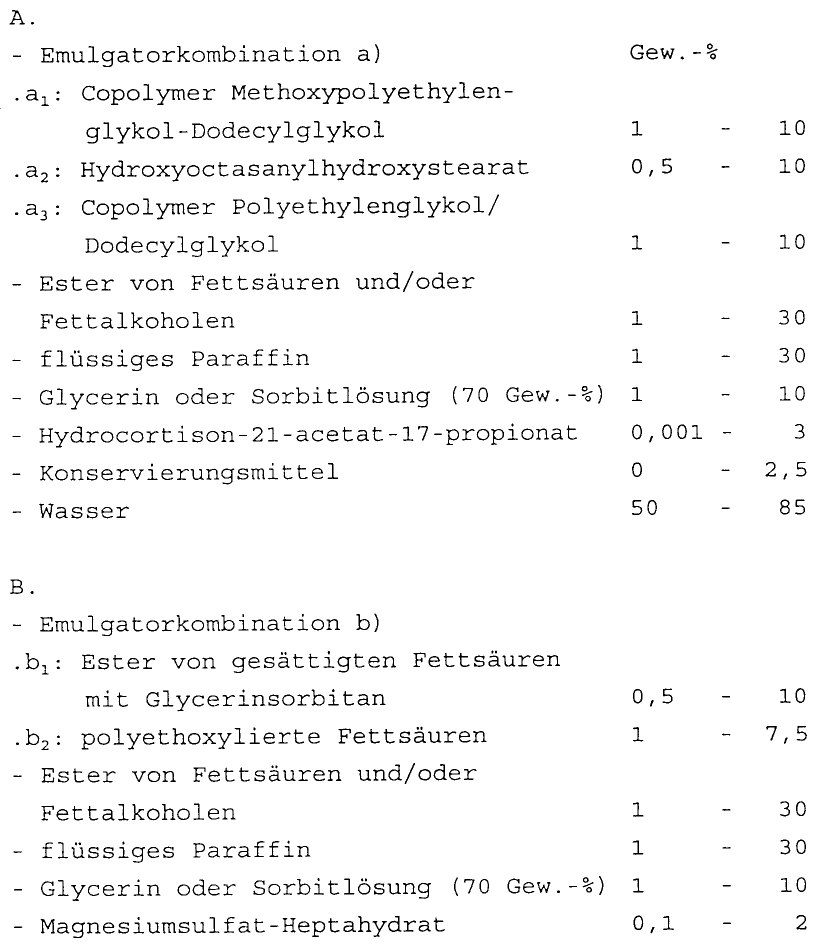 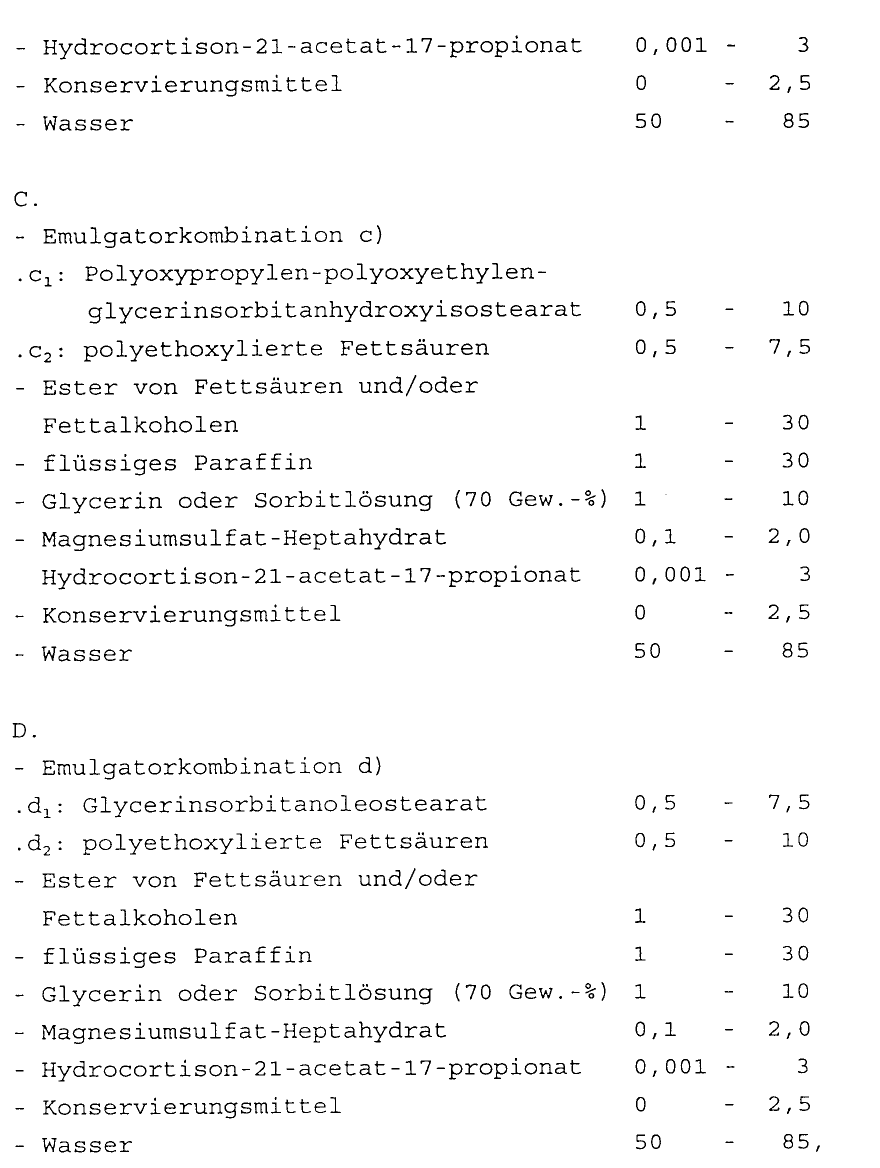 wobei jede der Zusammensetzungen A bis D gegebenenfalls Zusatzstoffe und außer der angegebenen Emulgatorkombination einen oder mehrere der anderen genannten Bestandteile a₁ bis d₂ oder eine oder mehrere obengenannte Emulgatorkombinationen a) bis d) enthalten können.

2. Lotionen nach Anspruch 1, die die folgenden Bestandteile und optionalen Hilfsstoffe enthalten:

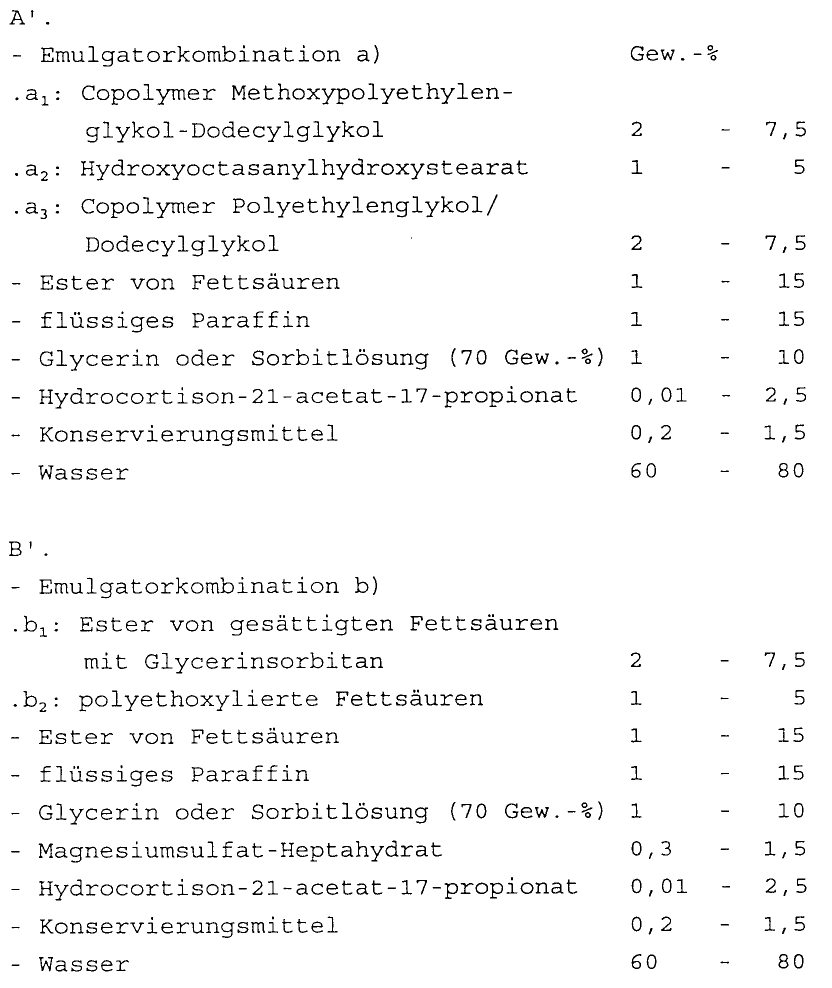

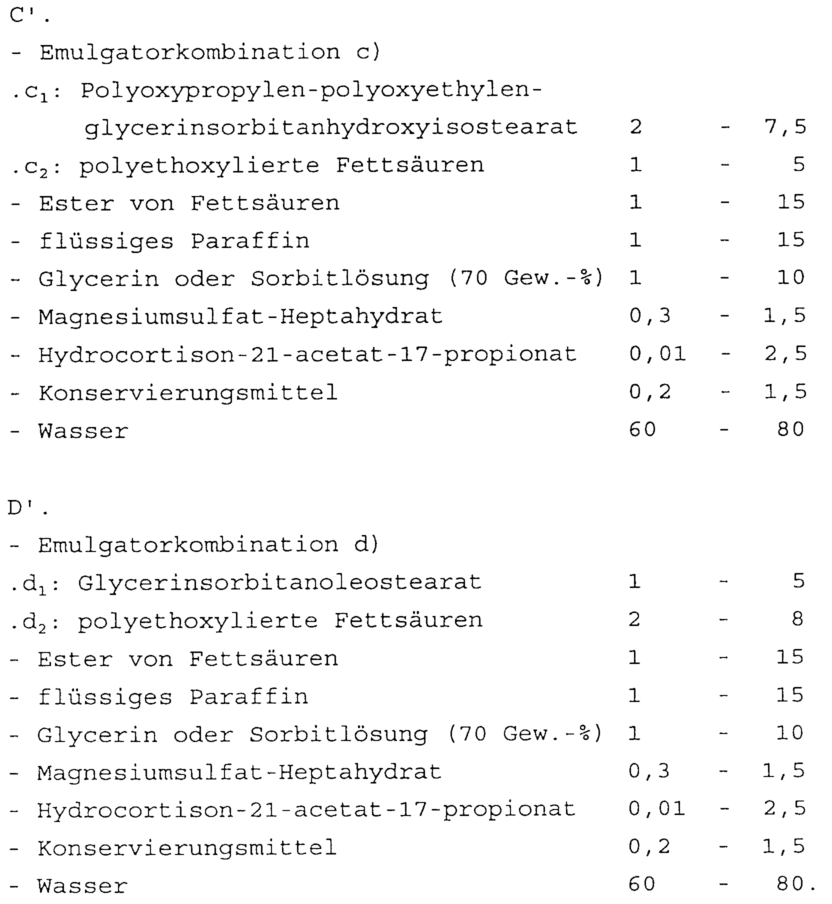

3. Lotionen nach Anspruch 1 oder 2, dadurch gekennzeichnet, daß sie 0,0025 bis 0,2 Gew.-% Hydrocortison-21-acetat-17-α-propionat (Hydrocortisonaceponat) enthalten.

4. Lotionen nach Anspruch 1, die die folgende Zusammensetzung aufweisen:
| | Gew.-% |
|---|---|
| Ester einer gesättigten Fettsäure mit Glycerinsorbitan (Arlacel 986) | 1,5 |
| polyethoxylierte Fettsäuren (Arlacel 989 oder Cremophor WO 7) | 3,0 |
| Isopropylmyristat | 5,0 |
| flüssiges Paraffin | 11,0 |
| 1,3-Butylenglykol | 3,0 |
| Magnesiumsulfat-Heptahydrat | 0,5 |
| Hydrocortison-21-acetat-17-propionat | 0,127 |
| Benzylalkohol | 1,0 |
| entmineralisiertes Wasser | ad 100,0. |
